(19) Europäisches Patentamt

European Patent Office

Office européen des brevets

(11) Publication number: **0 343 286 B1**

# EUROPEAN PATENT SPECIFICATION

(45) Date of publication of patent specification: **19.08.92**   (51) Int. Cl.⁵: **A61M 5/14**

(21) Application number: **88201044.0**

(22) Date of filing: **24.05.88**

(54) **Volumetric pump for parenteral perfusion.**

(43) Date of publication of application:
**29.11.89 Bulletin 89/48**

(45) Publication of the grant of the patent:
**19.08.92 Bulletin 92/34**

(84) Designated Contracting States:
**AT BE CH DE ES FR GB GR IT LI LU NL SE**

(56) References cited:
**AT-B- 196 550**
**DE-C- 1 548 992**

**JOURNAL OF THORACIC AND CARDIOVAS-
CULAR SURGERY vol.57, May 1969, pages
675-678, Philadelphia; JOHNSON et al.:" Con-
tinuous flush of arterial pressure-recording
catheters"**

**BIOMEDIZINISCHE TECHNIK vo. 26, no. 10,
Oktober 1981, Berlin, pages 244-248; L. AUER
ET AL.:"Beschreibung eines Infusions-
Dosis-Kontrollgerätes"**

(73) Proprietor: **Ruano Marco, Miguel
Rue Dr. Moliner 2
Valencia(ES)**

(72) Inventor: **Ruano Marco, Miguel
Rue Dr. Moliner 2
Valencia(ES)**

(74) Representative: **Claeys, Pierre et al
Bureau Gevers rue de Livourne 7 Bte 1
B-1050 Bruxelles(BE)**

Rank Xerox (UK) Business Services

EP 0 343 286 B1

## Description

The invention relates to an apparatus for parenteral perfusion of a liquid as indicated in the preamble of claim 1.

The development of hospital medicine has in the past two decades resulted in an extraordinary increase in the use of solutions for parenteral administration. The traditional systems with a drip metering chamber and flow regulator by means of metal plates or wheels producing variable degrees of constriction in the diameter of the tube are highly unsatisfactory with regard to flow accuracy, constancy and range, having no mechanisms for sensing the end of the drip, obstruction of the system or other faults. To solve these problems, which are especially important in intravenous administration of drugs, various types of infusion pumps have been appearing on the market, which control flow by various methods : peristaltic, syringes, chambers with compressible diaphragm, etc. These all necessitate changing the conventional lines or adding a second component to the line described above, which it is recommended be changed every 24 hours for each patient, in order to prevent infection.

A continuous flushing system is disclosed in an article of Johnson et al : Continuous flush of arterial pressure-recording catheters, Journal of Thoracic and Cardiovascular Surgery, vol. 57, 1969, p. 675-678. Said system comprises a bottle with gas under pressure which supplies a pressure infusion bag with said gas, a plastic plasma transfer bag being lodged within said pressure infusion bag, and a marine bore glass tubing controlling the flow resistance in the outlet tube of the plasma transfer bag and allowing a constant flow rate in said tube.

Bearing in mind that one of the commonest forms of solution for parenteral administration is presented in collapsible containers which make it possible to develop a system which, by acting directly on the collapsible bag of fluid to be administered intravascularly, will achieve volumetric control of perfusion without the addition of any items to the conventional system, the applicant has designed the incorporation of the volumetric pump described here, in order to achieve total safety and provide optimum working conditions by the use of this pump. Preferably, the apparatus according to the invention does not require the substitution or addition of auxiliary pieces of equipment to the conventional types of drip systems normally used.

In order to solve these problems, there is provided according to the invention an apparatus as indicated in the preamble of claim 1 and having the features of the characterizing portion of said claim. Other embodiments of the invention are provided in subclaims 2 to 5.

In principle, the system consists basically of a rigid chamber inside which the hermetically sealed bag containing the perfusion fluid is placed and which by means of a liquid or gaseous interphase forms a common volume with another independant chamber, with which it remains in communication and which acts as a pump or impeller unit.

It works on the principle of forming a liquid interphase which is not in direct contact with the bag containing the fluid, as it has a rigid chamber which holds the liquid in a watertight compartment by means of a highly distensible membrane which acts directly on the external walls of the bag of perfusion solution ; this chamber can be opened out into two sections like a book. The membrane described is situated inside the internal walls of the chamber, and the perfusion bag is placed on this. The chamber is then hermetically sealed and all possible residual air is sucked out by means of suction applied to an air vent by means of a valve in order to provide a complete seal.

On completion of all the actions described above, the perfusion fluid and the impeller liquid are separated by the walls of the bag and the membrane in the chamber and form the total practical volume of the rigid chamber, and as a consequence, change of volume introduced in the pump cavity are transmitted in their entirety, immediately and in linear form to the external chamber, and consequently to the perfusion liquid, an equivalent volume of which flows out through the connection line to the patient.

There are many possible variations for application of the same working principle as that described for this invention, for example : placing the bag of perfusion solution in direct contact with the impeller liquid without a dividing membrane. A gate valve would be located between the compartments of the rigid chamber and the pressure chamber. After the bag is placed inside the compartment of the rigid chamber and the latter sealed, communication with the pressure chamber opens to allow the liquid to flow through, the air being exhausted through an air vent provided for that purpose in the upper part.

Perfusion flow rate is controlled by a pump, which may in turn be actuated by some sort of mechanism, either of piston, syringe or peristaltic type, or a receptacle with compressible diaphragm, etc., which would be equipped with the necessary timers to establish the selected flow.

To ensure greater precision, any of the possible variations could incorporate a servomechanism, which, based on counting the drips of fluid in the chamber in the patient's line, could establish determination of the volume effectively administered to the patient ; this information could be used for regulating operation of the pump, by correcting any existing deviations from the pre-set flow. Such counting of drips is disclosed for example in L. Auer et al, Beschreibung eines Infusions-Dosis-Kontrollgerätes, Biomedizinische Technik, Vol. 26 (1981) n° 10, p. 244-248.

The system described here will include the usual warnings and controls for intravascular perfusion pumps, e.g. for obstruction of the system, bubbles in the perfusion line, etc., but unlike the existing systems it enables the administered flow to be controlled without substitution or action on the system or conventional drip line.

Essentially, the volumetric pump for parenteral perfusion of solutions without the need to replace or add complementary components to the conventional drip systems currently used, covered by this Invention Patent, enables administration of perfusion fluid, by compression of the bag containing the fluid, by means of a pump or similar device controlling flow rate and administration volume as required.

The system described, consisting of a volumetric pump for parenteral perfusion, unlike existing systems, enables the administered flow to be controlled without changing or working on the system or conventional drip line, or replacing or adding new items to it, and this modified system acts as a pumping chamber ; in this way, the system described is the only one in which action of the pump is proposed on the bag containing the perfusion fluid, not just by the application of pressure, but by controlling flow and administration volume as required.

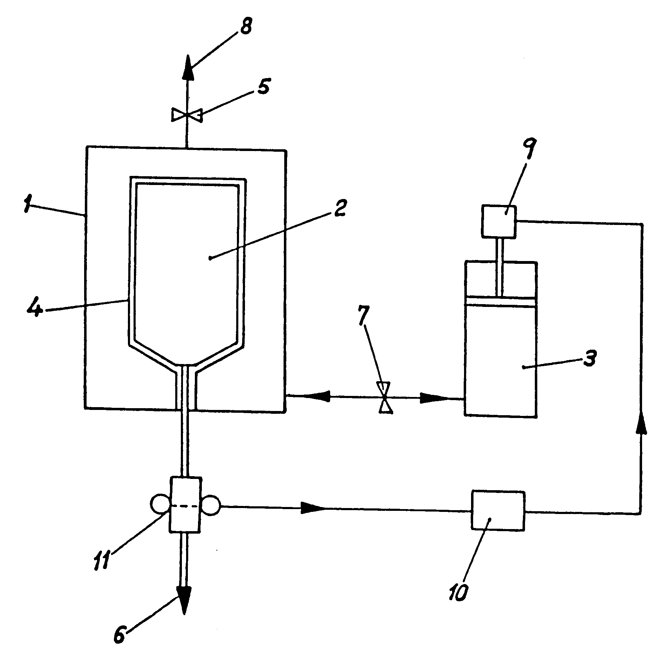

For better comprehension of the general characteristics described above, a sheet of drawings is attached hereto, illustrating graphically a practical example of a version of the volumetric pump for parenteral perfusion covered by this application for patent, and it is hereby stated that in view of the highly informative nature of the drawing in question on the attached sheet, it should be studied in the broadest possible light and considered as not restrictive in any way.

The single figure on the attached sheet is a general arrangement diagram of all the components of the system, the interconnections between them and their functions.

Still referring to the attached drawing, it should be noted that it includes reference numerals keyed to the descriptions of its characteristics and operation described below, to assist in their immediate location ; these consist basically of a rigid chamber (1) inside which is incorporated the bag (2) of perfusion fluid, and by means of a liquid or gaseous interphase a common volume is formed between chamber (1) and another chamber (3), with which it remains in communication, chamber (3) acting as a pump or fluid impeller unit.

The system works on the basic principle of formation of a liquid interphase without direct contact with the bag (2) containing the fluid, with the rigid chamber (1) holding the liquid in a watertight compartment by means of a dividing membrane (4) with high distension capacity, which acts directly on the walls of the bag (2) containing the perfusion solution.

The chamber (1) can be opened up into two sections like a book ; there is a membrane (4) inside its internal walls on which the perfusion bag (2) is placed, chamber (1) is then hermetically sealed and the residual air sucked out by application of suction to the air vent (8), which is fitted with a valve (5) to ensure a complete seal.

From then on, the perfusion fluid and the impeller liquid, separated by the walls of the bag (2) and the membrane (4) of the rigid chamber (1) represent the total practical volume of the rigid chamber ; consequently, changes in volume introduced in the pump cavity (3) are transmitted immediately and in their entirety in linear form to the chamber (1) and consequently to the perfusion liquid, an equivalent volume of which will flow out through the connection line (6) to the patient.

A gate valve (7) is fitted in the communication between the compartments (1) and (3) ; in this way, when the bag (2) has been placed inside the compartment (1) and the latter sealed, the communication between compartments (1) and (3) is opened to allow the impeller liquid to flow through, included air being expelled through the air vent (8) provided for that purpose at the top ; there are many possible variants permitting application of the same working principle as that covered by this invention, for example: placing the bag (2) containing the perfusion solution in direct contact with the impeller liquid, with no separating chamber.

Perfusion rate is controlled by means of the pump (3), which may be actuated by any type of mechanism, either piston or syringes as shown in the attached diagram, or peristaltic, or a receptacle with compressible diaphragm, etc., all of which would be equipped with the necessary timers (9) for establishing the selected flow.

To ensure greater accuracy, any of the methods used could incorporate a servomechanism (10) which, on the basis of counting the fluid drips in the chamber of the patient's line (11), could establish determination of the volume effectively administered to the patient, this information being used to regulate operation of the pump (3) by correcting possible deviations from the pre-set flow.

In the belief that each and every one of the component parts of this volumetric pump for parenteral perfusion have been fully described, it now only remains to mention the possibility of construction of the different components in a variety of materials, sizes and forms, as well as the possibility of introduction in its layout of such design variations as prove expedient in practice, always providing these cannot change the essential points covered by this Invention Patent.

**Claims**

1. Apparatus for parenteral perfusion of a liquid to a patient from a bag (2) of perfusion liquid having an outlet tube (6), comprising

   a housing chamber (1) which receives the perfusion bag (2) and defines an enclosed space surrounding said bag, the outlet tube (6) extending from said housing chamber (1),

   a pumping chamber (3) with which said space of the housing chamber (1) remains in communication,

   and connection means (7) between said pumping chamber (3) and said housing chamber (1) for filling said space with pressurized fluid received from said pumping chamber (3) for applying pressure on said bag (2) to produce a passage of perfusion liquid from said bag through said outlet tube (6),

   characterized in that

   the housing chamber (1) is a rigid chamber provided with means (3, 8) for evacuating said space and with a valve (5) to ensure airtightness,

   and in that

   the apparatus comprises means (9, 10) for controlling that rate of fluid flow from said pumping chamber (3) into said space to control the perfusion flow rate through the outlet tube (6), and optionally sensor means (11) for sensing the amount of liquid administered to the patient through the outlet tube (6) and operatively connected to said means (9, 10) for controlling the rate of fluid flow from said pumping chamber (3) into said space.

2. Apparatus according to claim 1, wherein said rigid housing chamber (1) includes a membrane (4) with high distension capacity which acts directly on the bag (2) of perfusion liquid and which defines a fluidtight compartment within the rigid housing chamber (1).

3. Apparatus according to claim 1 or 2, wherein said rigid housing chamber (1) comprises two sections able to be opened like a book for insertion of said perfusion bag (2) in said space between said two sections.

4. Apparatus according to any one of claims 1 to 3, wherein said means (9, 10) for controlling the rate of fluid flow are mechanisms controlling the pumping chamber (3) of piston, syringe or peristaltic type, preferably equipped with timers (9) to establish the selected flow.

5. Apparatus according to any one of claims 1 to 4, wherein said sensor means (9, 10) comprise a servomechanism (10) which on the basis of counting the drops of fluid (in 11) passing through the outlet tube (6) enables the determination of the volume effectively administered to the patient and the regulation of said means (9, 10) for controlling the rate of fluid flow.

6. Apparatus according to any one of claims 1 to 5, wherein said pressurized fluid received from said pumping chamber (3) into said space is a liquid.

**Patentansprüche**

1. Vorrichtung für eine parenterale Perfusion einer Flüssigkeit an einen Patienten aus einem Beutel (2) einer Perfusionsflüssigkeit, der ein Auslaßrohr (6) aufweist, mit

   einer Aufnahmekammer (1), die den Perfusionsbeutel (2) aufnimmt und einen umschlossenen Raum definiert, der den Beutel umgibt, wobei sich das Auslaßrohr (6) aus der Aufnahmekammer (1) heraus erstreckt,

   eine Pumpenkammer (3), mit der der Raum der Aufnahmekammer (1) in Verbindung steht, und eine Verbindungseinrichtung (7) zwischen der Pumpenkammer (3) und der Aufnahmekammer (1) zum Füllen des Raumes mit einem unter Druck stehenden Fluid, das aus der Pumpenkammer (3) erhalten wird zum Anlegen von Druck auf den Beutel (2), um einen Durchlaß der Perfusionsflüßeigkeit aus dem Beutel durch das Auslaßrohr (6) zu bewirken, **dadurch gekennzeichnet,** daß

   die Aufnahmekammer (1) eine steife Kammer ist, die mit einer Einrichtung (3, 8) zum Auspumpen des Raumes und mit einem Ventil (5) versehen ist, um Luftdichtheit abzusichern,

   und daß

   die Vorrichtung Einrichtungen (9, 10) umfaßt zum Steuern der Fluidgeschwindigkeit, die aus der Pumpenkammer (3) in den Raum strömt, um die Perfusionsströmungsgeschwindigkeit durch das Auslaßrohr (6) zu steuern, und eine frei wählbare Sensoreinrichtung (11) umfaßt zum Abtasten der dem Patienten zu verabreichenden Flüssigkeitsmenge durch das Auslaßrohr (6) und die wirksam mit der Einrichtung (9, 10) verbunden ist zum Steuern der Fluidströmungsgeschwindigkeit aus der Pumpenkammer (3) in den Raum.

2. Vorrichtung nach Anspruch 1, wobei die steife Aufnahmekammer (1) eine Membran (4) mit hohem Dehnungsvermögen aufweist, die direkt auf den Beutel (2) der Perfusionsflüssigkeit wirkt und die eine fluiddichte Kammer in der steifen Aufnahmekammer (1) definiert.

3. Vorrichtung nach Anspruch 1 oder 2, wobei die steife Gehäusekammer (1) zwei Abschnitte umfaßt, die wie ein Buch zu öffnen sind zum Einsetzen des Perfusionsbeutels (2) in den Raum zwischen den beiden Abschnitten.

4. Vorrichtung nach einem der Ansprüche 1 bis 3, wobei die Einrichtungen (9, 10) Zum Steuern der Fluidströmungsgeschwindigkeit ein Mechanismus ist, der die Pumpenkammer (3) steuert, die ein Kolben-, Spritzen- oder Schlauchpumpentyp ist, und die vorzugsweise mit einem Zeitgeber (9) ausgestattet ist, um die ausgewählte Strömung herzustellen.

5. Vorrichtung nach einem der Ansprüche 1 bis 4, wobei die Sensoreinrichtung (9, 10) einen Servomechanismus (10) umfaßt, der auf der Basis des Zählens der Tropfen des Fluids (in 11), das durch das Auslaßrohr (6) hindurchtritt, die Bestimmung des Volumens, das dem Patienten wirksam verabreicht wird und die Regulierung der Einrichtung (9, 10) zum Steuern der Fluidströmungsgeschwindigkeit ermöglicht.

6. Vorrichtung nach einem der Ansprüche 1 bis 5, wobei das unter Druck stehende Fluid, das in dem Raum aus der Pumpenkammer (3) erhalten wird, eine Flüssigkeit ist.

**Revendications**

1. Dispositif de perfusion par voie parentérale d'un liquide à un patient à partir d'un sac (2) de liquide de perfusion présentant un tube de sortie (6), comprenant

une chambre de logement (1) qui reçoit le sac de perfusion (2) et qui forme un espace fermé entourant le sac, le tube de sortie (6) faisant saillie à partir de cette chambre de logement (1),

une chambre de pompage (3) avec laquelle l'espace de la chambre de logement (1) reste en communication, et

des moyens de liaison (7) entre la chambre de pompage (3) et la chambre de logement (1) pour remplir ledit espace par du fluide sous pression reçu de la chambre de pompage (3) pour appliquer une pression sur ce sac (2) en vue de produire un passage du liquide de perfusion à partir du sac à travers ledit tube de sortie (6),

caractérisé en ce que

la chambre de logement (1) est une chambre rigide pourvue de moyens (3, 8) pour mettre ledit espace sous vide et d'une vanne (5) pour assurer une étanchéité à l'air,

et en ce que

le dispositif comprend des moyens (9, 10) pour contrôler cette vitesse de l'écoulement de fluide provenant de la chambre de pompage (3) dans ledit espace en vue de contrôler la vitesse d'écoulement de la perfusion à travers le tube de sortie (6), et éventuellement des moyens détecteurs (11) pour détecter la quantité du liquide administré au patient par le tube de sortie (6) et reliés de manière fonctionnelle auxdits moyens (9, 10) de contrôle de la vitesse de l'écoulement du fluide depuis ladite chambre de pompage (3) dans ledit espace.

2. Dispositif suivant la revendication 1, caractérisé en ce que la chambre de logement rigide (1) contient une membrane (4) présentant une haute capacité d'extension qui agit directement sur le sac (2) du liquide de perfusion et qui forme un compartiment étanche aux fluides à l'intérieur de la chambre de logement rigide (1).

3. Dispositif suivant l'une des revendications 1 et 2, caractérisé en ce que la chambre de logement rigide (1) comprend deux sections capables d'être ouvertes comme un livre pour l'introduction dudit sac de perfusion (2) dans l'espace entre les deux sections.

4. Dispositif suivant l'une quelconque des revendications 1 à 3, caractérisé en ce que les moyens (9, 10) pour contrôler la vitesse d'écoulement du fluide sont des mécanismes qui contrôlent la chambre de pompage (3) de type piston, seringue ou péristaltique, ces mécanismes étant de préférence équipés de compteurs de temps (9) pour établir l'écoulement sélectionné.

5. Dispositif suivant l'une quelconque des revendications 1 à 4, caractérisé en ce que lesdits moyens détecteurs (9, 10) comprennent un servomécanisme (10) qui, sur la base du comptage des gouttes de fluide (en 11) passant à travers le tube de sortie (6), permet la détermination du volume effectivement administré au patient et le réglage des moyens (9, 10) de contrôle de la vitesse de l'écoulement de fluide.

6. Dispositif suivant l'une quelconque des revendications 1 à 5, caractérisé en ce que le fluide sous pression reçu depuis la chambre de pompage (3) dans ledit espace est un liquide.